Europäisches Patentamt

European Patent Office

Office européen des brevets

(19)

(11) Veröffentlichungsnummer: **0 165 454**
**B1**

(12) # EUROPÄISCHE PATENTSCHRIFT

(45) Veröffentlichungstag der Patentschrift: **23.01.91**

(51) Int. Cl.⁵: **A 61 K 7/16**

(21) Anmeldenummer: **85105826.3**

(22) Anmeldetag: **11.05.85**

(54) **Zahnpflegemittel für hypersensible Zähne.**

(30) Priorität: **18.05.84 DE 3418427**

(43) Veröffentlichungstag der Anmeldung:
**27.12.85 Patentblatt 85/52**

(45) Bekanntmachung des Hinweises auf die
Patenterteilung:
**23.01.91 Patentblatt 91/04**

(84) Benannte Vertragsstaaten:
**AT BE CH DE FR GB IT LI LU NL SE**

(56) Entgegenhaltungen:
**DE-A-2 134 862**
**US-A-4 342 741**

(73) Patentinhaber: **Württembergische Parfümerie-
Fabrik GmbH
Schillerstrasse 21-27
D-7332 Eislingen/Fils 16 (DE)**

(72) Erfinder: **Scheller, Hans-Ulrich, Dr.
Vogelgartenstrasse 45
D-7332 Eislingen/Fils (DE)**

(74) Vertreter: **Werner, Hans-Karsten, Dr. et al
Deichmannhaus am Hauptbahnhof
D-5000 Köln 1 (DE)**

**Die Akte enthält technische Angaben, die nach
dem Eingang der Anmeldung eingereicht
wurden und die nicht in dieser Patentschrift
enthalten sind.**

EP 0 165 454 B1

Courier Press, Leamington Spa, England.

**Beschreibung**

Gegenstand der vorliegenden Erfindung ist ein Zahnpflegemittel für hypersensible Zähne, enthaltend einen Apatit mit einer mittleren Teilchengröße unter 10 μm sowie ggf. ein Lokalanästhetikum.

Aus der US—A—4 342 741 sind Zahnpasten bekannt, die einen Hydroxyapatit mit einer mittleren Teilchengröße von 2 μm enthalten; die Rezepturen enthalten jedoch weiters alle mindestens ein lösliches Mineralsalz.

Die DE—C—21 34 862 beschreibt ebenfalls derartige Zahnpflegemittel. Die Rezepturen enthalten jedoch zusätzlich eine osmotisch wirksame Salzkombination aus Natriumhydrogencarbonat, Natriumchlorid, Magnesiumcarbonat, Magnesiumchlorid, Kaliumsulfat etc.

Aus der US—A—31 22 483 bekannt, Zahnpflegemitteln Strontiumchlorid zuzusetzen, da dies die Reizbarkeit des Nerves in der Pulpa beeinflussen soll. Eine Beeinflussung der Pulpa ist zahnmedizinisch wenig erwünscht und kann andere negative Folgen hervorrufen.

Ein weiteres Mittel zur Behandlung von Hypersensibilitäten ist die lokale Anwendung von Aminfluoridlösungen. Diese Behandlung hat jedoch den Nachteil, daß sie nur in der zahnärztlichen Praxis durchgeführt werden kann und bei der üblichen Zahnpflege nur von kurzer Dauer ist.

Umfangreiche experimentelle Arbeiten mit dem Ziel, das Handelsprodukt gemäß DE—C—21 34 862 zu verbessern, führten zu dem überraschenden Ergebnis, daß der Zusatz von Strontiumchlorid zu keiner Verbesserung der Wirkung führte. Stattdessen wurde festgestellt, daß die Wirksamkeit dadurch in erheblichem Maß verbessert werden konnte, daß alle löslichen Mineralsalze weggelassen wurden und als alleinige kristalline und polierende Substanz ein Apatit eingesetzt wurde, dessen mittlere Teilchengröße unter 8 μm liegt. Ein derartiger Apatit hat obendrein einen wesentlich geringeren Abriebwert (gemessen als RDA; vgl. Radioactive Dentine Abrasion, J. J. Hefferren, J. Dental Research 55, Nr. 4, 563—573 (1976)) von weniger als 30. Die Menge an Apatit mußt dabei höher liegen als bei dem Handelsprodukt, nämlich bei mindestens 15 Gew.-% anstelle der bisher eingesetzten 9%.

Diese Ergebnisse waren nicht vorhersehbar und können auch nachträglich nicht ohne weiteres erklärt werden. Die Befunde deuten jedoch darauf hin, daß es sich um einen Poliereffekt handelt. Beim Polieren bildet sich nämlich stets an der Oberfläche eine zusammenhängende, glasartige, glänzende Schicht, die einerseits aus dem Grundstoff des polierten Materials und andererseits aus dem Poliermittel besteht. Beim Polieren werden diese beiden mechanisch eng miteinander verbunden und verschweißt. Das Poliermittel wird somit mechanisch zwischen die mikroskopischen, mikrokristallinen bis atomaren Unebenheiten und Rauhigkeiten der Oberfläche eingerieben und mit dieser durch lokale Überhitzung und Eutektikumsbildung verschmolzen. Übertragen auf die Oberfläche des Zahnes könnte dies bedeuten, daß bei der Behandlung mit großen Mengen des feinteiligen Apatits dieser in die offenliegenden Dentinkanälchen gedrückt wird. Das es sich um das gleiche Material handelt wie die Hartsubstanz des Zahnes, können die vorhandenen Schäden ausheilen, sofern keine störenden Fremdmineralien den Verschmelzungsprozess unterbinden. Weiterhin muß die Menge an angebotenem Apatit groß genug sein und verhindert werden, daß durch andere abrasive Stoffe die polierte Fläche wieder aufgerissen und zerstört wird.

Wietere Untersuchungen haben gezeigt, daß insbesondere bei Teilchengrößen des Apatits unter 4 μm eine stärkere Remineralisation eintritt. Schon bei 20-facher Anwendung in vitro wurde eine Verkleinerung der Durchmesser der Dentinkanäle von ca. 50% beobachtet.

Unabhängig von dieser nachträglichen theoretischen Erklärung steht fest, daß nur bei Erfüllung aller erfindungsgemäßen Bedingungen der gewünschte Effekt ein tritt.

Gegenstand der vorliegenden Erfindung ist somit ein Zahnpflegemittel für hypersensible Zähne, enthaltend einen Apatit mit einer mittleren Teilchengröße unter 10 μm, sowie ggf. ein Lokalanästhetikum, dadurch gekennzeichnet, daß es als alleinige kristalline und polierende Substanz 15 oder mehr Gew.-% des Apatits mit einer mittleren Teilchengröße unter 8 μm und einem Abriebwert (RDA) von weniger als 30 enthält, jedoch keine weiteren löslichen Mineralsalze.

Als Apatit kann von allem Hydroxyapatit, Fluorapatit oder auch ein emisch derselben eingesetzt werden. Wichtig ist, daß die Menge, die Korngröße und der Abriebwert eingehalten werden und eine weiteren löslichen Mineralsalze ihren störenden Effekt ausüben können.

Als nicht störend hat sich allein amorphe Kieselsäure erwiesen, die in Mengen bis zu 10 Gew.-% zugegeben werden kann, ohne die Wirksamkeit negativ zu beeinflussen.

Selbstverständlich können die erfindungsgemäßen Zahnpflegemittel zusätzlich ein Lokalanästhetikum enthalten wie Benzocain, p-Aminobenzoesäureethylester. Weiterhin enthalten die erfindungsgemäßen Zahnpflegemittel neben Wasser und Glycerin Netz- und Schaummittel, Geschmacks- und Aromastoffe. Gewünschtenfalls können auch alle anderen Zusätze verwendet werden, sofern es sich nicht um lösliche Mineralsalze handelt.

Der erfindungsgemäß verwendete Apatit mit einer mittleren Teilchengröße unter 8 μm und einem Abriebwert (RDA) von weniger als 30 ist der Fachwelt als sogenannter amorpher Hydroxyapatit, Fluorapatit oder ein Gemisch derselben bekannt. Das Schüttgewicht beträgt vorzugsweise weniger als 180 g/l und liegt meist im Bereich von etwa 150 g/l.

In den nachfolgenden Beispielen und Versuchen ist das erfindungsgemäße Zahnpflegemittel näher beschrieben.

Beispiel 1
Zahncreme (Rezeptur A)

| | |
|---|---|
| Amorphe Kieselsäure Aerosil® 200 | 2,40% |
| Carboxymethylcellulose | 1,00% |
| Natriumlaurylsulfat | 2,75% |
| Glycerin (99 %ig) | 20,80% |
| Netzmittel (Hostapon® KTW) | 0,90% |
| p-Hydroxybenzoesäuremethylester-Na | 0,20% |
| Saccharin-Na | 0,25% |
| Tricalciumhydroxyapatit | 17,00% |
| Wasser | 50,699% |
| S-Erythrosin 76 E 127 | 0,001% |
| Aroma | 1,50% |
| Propylenglykol | 2,50% |

Die aus diesen Bestandteilen bestehende Zahnpasta wurde im Abrasionstest (RDA) getestet und ergab eine Abrasion von 24.

Klinische Untersuchungen dieser Zahnpasta ergaben nach 3 bis 8 Tagen bereits eine deutliche Besserung und nach spätestens 15 Tagen eine vollständige Schmerzfreiheit bei allen Benutzern.

Vergleichsversuche

Zum Vergleich wurden die folgenden Rezepturen B bis G hergestellt:

| | B | C | D | E | F | G |
|---|---|---|---|---|---|---|
| Amorphe Kieselsäure Aerosil® 200 | 3,00 | 2,85 | 10,00 | 3,00 | 2,40 | 2,40 |
| Carboxymethylcellulose | 1,00 | 1,00 | 1,00 | 1,00 | 1,00 | 1,00 |
| Natriumlaurylsulfat | 2,75 | 2,75 | 2,75 | 2,75 | 2,75 | 2,75 |
| Glycerin (99 %ig) | 20,80 | 20,80 | 20,80 | 20,80 | 20,80 | 20,80 |
| Netzmittel (Hostapon® KTW) | 0,90 | 0,90 | 0,90 | 0,90 | 0,90 | 0,90 |
| p-Hydroxybenzoesäuremethylester-Na | 0,20 | 0,20 | 0,20 | 0,20 | 0,20 | 0,20 |
| Saccharin-Na | 0,25 | 0,25 | 0,25 | 0,25 | 0,25 | 0,25 |
| Tricalciumhydroxyapatit | — | — | — | 9,00 | 17,00 | 17,00 |
| Wasser | 49,499 | 49,499 | 60,99 | 49,499 | 44,699 | 44,699 |
| S-Erythrosin 76 E 127 | 0,001 | 0,001 | 0,001 | 0,001 | 0,001 | 0,001 |
| Aroma | 1,50 | 1,50 | 1,50 | 1,50 | 1,50 | 1,50 |
| Propylenglykol | 2,50 | 2,375 | 2,50 | 2,375 | 2,50 | 2,50 |
| Kaliumsulfat | 0,175 | — | — | 0,175 | — | — |
| Magnesiumcarbonat | 1,25 | — | — | 1,25 | — | — |
| Natriumhydrogencarbonat | 6,25 | — | — | 6,25 | — | — |
| Natriumchlorid | 0,175 | — | — | 0,175 | — | — |
| Titandioxyd | 0,75 | 0,75 | — | 0,75 | — | — |
| Dicalciumphosphat×2 $H_2O$ | 9,00 | 17,00 | — | — | — | — |
| Benzocain | — | 0,125 | — | 0,125 | — | — |
| Strontiumchlorid×6 $H_2O$ | — | — | — | — | 6,00 | 5,00 |
| Cetylaminhydrofluorid | — | — | — | — | — | 1,00 |

Die Rezeptur E entspricht dem Handelsprodukt gemäß DE—C—21 34 862. Die Rezeptur B entspricht diesem Handelsprodukt, jedoch ohne Hydroxylapatit. Die Rezeptur C entpsircht der Rezeptur B, jedoch unter Zusatz von Benzocain. Die Rezeptur D ist ein Placebo ohne jeglichen Wirkstoff. Die Rezepturen F und G entsprechen der erfindungsgemäßen Rezeptur A, enthalten jedoch zusätzlich Strontiumchlorid×6 $H_2O$ oder Cetylaminhydrofluorid.

Die klinische Untersuchung ergab, daß die Wirksamkeit des Handelsproduktes sowie einiger anderer Rezepturen zwar besser war als die des Placebo, jedoch wesentlich geringer als bei der erfindungsgemäßen Rezeptur A.

Abrasionsteste

Die Abrasion (RDA) wurde gemessen von den Rezepturen A und F sowie einigen bekannten Handelsprodukten. Dabei wurde festgestellt, daß die Abrasion der Rezeptur F bereits bei 61 lag. Die Werte für die Handelsprodukte Blend-a-med, Lacalut, Theramed und Causamed lag zwischen 85 und 95.

**Patentansprüche**

1. Zahnpflegemittel für hypersensible Zähne, enthaltend einen Apatit mit einer mittleren Teilchengröße unter 10 µm, sowie ggf. ein Lokalanästhetikum, dadurch gekennzeichnet, daß es als alleinige kristalline und polierende Substanz 15 oder mehr Gew.-% des Apatits mit einer mittleren Teilchengröße unter 8 µm und einem Abriebwert (RDA) von weniger als 30 enthält, jedoch keine weiteren löslichen Mineralsalze.

2. Zahnpflegemittel gemäß Anspruch 1, dadurch gekennzeichnet, daß der Apatit en Hydroxy- oder Fluorapatit ist.

3. Zahnpflegemittel gemäß Ansprüchen 1 oder 2, dadurch gekennzeichnet, daß die mittlere Teilchengröße des Apatits unter 4 µm liegt.

**Revendications**

1. Dentifrice pour dents hypersensibles, contenant une apatite d'une grosseur de particules moyenne inférieure à 10 µm, ainsi qu'éventuellement un anesthésique local, caractérisé en ce qu'il contient, comme seule substance cristalline et de polissage, 15% en poids, ou plus, de l'apatite présentant une grosseur de particules moyenne inférieure à 8 µm et une valeur d'abrasion (ARD) inférieure à 30, mais pas d'autres sels minéraux solubles.

2. Dentifrice selon la revendication 1, caractérisé en ce que l'apatite est une hydroxy- ou une fluorapatite.

3. Dentifrice selon la revendication 1 ou 2, caractérisé en ce que la grosseur moyenne des particules de l'apatite est inférieure à 4 µm.

**Claims**

1. A dentifrice for hypersensitive teeth containing an apatite having an average particle size of less than 10 µm and optionally a local anesthetic, characterized in that it contains an amount of 15% by weight or more of the apatite having an average particle size of less than 8 µm and an abrasion value (RDA) of less than 30 as the sole crystalline and polishing substance, however not any further soluble mineral salts.

2. The dentifrice according to claim 1, characterized in that the apatite is hydroxylapatite or fluoroapatite.

3. The dentifrice according to claims 1 or 2, characterized in that the average particle size of the apatite is less than 4 µm.